# EUROPÄISCHE PATENTANMELDUNG

(11) **EP 1 174 403 A2**
(43) Veröffentlichungstag der Anmeldung: **23.01.2002**
(21) Anmeldenummer: 01117385.3
(22) Anmeldetag: 18.07.2001
(51) Int. Cl.: C05F 3/00, C02F 1/66

(54) **Kalk-Ton-Suspension und ihre Verwendung**

(30) Priorität: 20.07.2000 DE 10035432
(71) Anmelder: Kalksteinwerk Vilshofen GmbH, 92286 Rieden (DE)
(72) Erfinder:
(74) Vertreter: Kuhnen & Wacker

(57) **Zusammenfassung**

Kalk-Ton-Suspension, insbesondere zur Gülleaufbereitung, mit einem Trockensubstanzgehalt von ca. 50 Gew.-%, wobei die Trockensubstanz feinkörnig vorliegt und die Bestandteile (in Gew.-%) enthält:

| | |
|---|---|
| Calciumcarbonat (CaCO₃) | 25 - 30, |
| Magnesiumcarbonat (MgCO₃) | 0,5 - 2, |
| Siliciumoxid (SiCO₂) | 8 - 12, |
| Aluminiumoxid (Al₂O₃) | 2 - 4, |
| Eisenoxid (Fe₂O₃) | 1-3. |

## Beschreibung

Die Erfindung betrifft eine Kalk-Ton-Suspension mit einem Trockensubstanzgehalt von ca. 50 Gew.-%. Kalk (Kalkstein).

Kalke (Kalksteine), die überwiegend aus Calciumcarbonat bestehen, lassen sich sehr vielseitig verwenden. In der Land- und Forstwirtschaft und im Landschaftsbau eignen sie sich z.B. als Bodenhilfsstoff zur Kalkung (Kalkdüngung) von Böden und zur Neutralisation übersäuerter Gewässer.

Aus natürlich vorkommenden Mineralgemischen aus Kalkstein und tonigen Bestandteilen lassen sich durch Naßtrennverfahren Mineralsuspensionen erhalten, die in der Bauwirtschaft und im Landschaftsbau als Dichtungsmaterialien, z.B. für Depotabdichtungen, eingesetzt werden.

Außerdem ist es auch bekannt, zur Gülleaufbereitung (zur Geruchsverringerung und zur Verbesserung von Lagerung und Transport) Gesteinsmehl oder feinkörnige Tonmineralien zu verwenden.

Erfindungsgemäß wird eine Kalk-Ton-Suspension aus Calciumcarbonat, Silicaten und Tonmineralien mit einem Trockensubstanzgehalt von ca. 50 Gew.-% bereitgestellt, die sich insbesondere sehr gut zur Aufarbeitung und Geruchsneutralisierung von Gülle, insbesondere von Rinder- und Schweinegülle, eignet.

Gegenstand der Erfindung ist eine Kalk-Ton-Suspension, insbesondere eine Kalk-Ton-Suspension zur Gülleaufbereitung, mit einem Trockensubstanzgehalt von ca. 50 Gew.-%, wobei die Trockensubstanz feinkörnig vorliegt und die Bestandteile (in Gew.-%) enthält:

| | |
|---|---|
| Calciumcarbonat (CaCO₃) | 25 - 30, |
| Magnesiumcarbonat (MgCO₃) | 0,5 - 2, |
| Siliciumoxid (SiO₂) | 8 - 12, |
| Aluminiumoxid (Al₂O₃) | 2 - 4, |
| Eisenoxid (Fe₂O₃) | 1-3. |

Bevorzugte Ausgestaltungen dieser Kalk-Ton-Suspension sind Gegenstand der Ansprüche 2 bis 5, bzw. 9 bis 13.

Vorzugsweise liegen 80 Gew.-% der erfindungsgemäßen Kalk-Ton-Suspension in einem Korngrößenbereich (Siebanalyse) von 0,001 mm bis 0,125 mm.

Die erfindungsgemäße Kalk-Ton-Suspension besitzt vorzugsweise einen pH-Wert von 7,5.

Die erfindungsgemäße Kalk-Ton-Suspension liegt vorzugsweise als Produkt vor, das durch Verarbeitung von natürlichem Kalkstein gewonnen wird.

Die erfindungsgemäße Kalk-Ton-Suspension kann aus einem natürlichen Kalkstein-Mineralgemisch hergestellt werden, indem man aus dem Mineralgemisch in einem Naßtrennverfahren die abschlämmbaren Kalk-, Silicat- und Tonpartikel gewinnt und die so erhaltene Aufschlämmung zu einer Kalk-Ton-Suspension mit einem Trockensubstanzgehalt von ca. 50 Gew.-% eindickt.

Gegenstand der Erfindung ist auch die Verwendung einer erfindungsgemäßen Kalk-Ton-Suspension in der Bauwirtschaft (z.B. als Dichtungsmaterial für Oberflächenabdeckungen, Deponieabdichtungen, zum Verpressen von Stollen und Kanälen, zur Herstellung von Ziegelprodukten), im Landschaftsbau (z.B. zur Hangbegrünung und für den Teichbau), in der Forstwirtschaft (zur Walddüngung), und insbesondere in der Wasserwirtschaft (zur Aufbereitung/Neutralisation von sauren Gewässem) und in der Landwirtschaft (Gülleaufbereitung und Bodenverbesserung).

Überraschenderweise wurde gefunden, daß sich eine erfindungsgemäße Kalk-Ton-Suspension hervorragend als Güllebehandlungsmittel zur Aufbereitung von Gülle, wie z.B. von Rinder- und Schweinegülle, eignet, und insbesondere zur Geruchsminderung, Verbesserung der Fließfähigkeit und Pflanzenverträglichkeit, zur Stickstoff- und Schwefelbindung (durch die Tonmineralien wird Ammoniumstickstoff gebunden). Ein Zusatz der erfindungsgemäßen Kalk-Ton-Suspension führt zu einer Abnahme des Geruches, sichtbaren Fermentierungsprozessen in der Güllegrube und zu einem besseren Abfließen von Pflanzen. Neben der Verbesserung der Fließfähigkeit kann durch einen Zusatz der erfindungsgemäßen Kalk-Ton-Suspension auch die pflanzenbauliche Wirkung verbessert werden.

Außerdem wurde gefunden, daß die Fließfähigkeit der Gülle in den Abflußkanälen der Ställe deutlich verbessert werden kann, wenn im Abstand von einigen Monaten eine mit der erfindungsgemäßen Kalk-Ton-Suspension behandelte Gülle in die Kanäle gegeben wird. Mit dieser Maßnahme werden die Fermentierungsvorgänge bereits im Stall angeregt.

Die erfindungsgemäße Kalk-Ton-Suspension liegt in dickflüssiger Form (Konsistenz) vor. Die Anlieferung kann durch entsprechend ausgestaltete Tankfahrzeuge erfolgen. Durch den Zusatz der erfindungsgemäßen Kalk-Ton-Suspension kann der sonst erforderliche Wasserzusatz zur Gülle verringert werden, wodurch Lagerraum eingespart wird und die Kosten für Transport und Ausbringung verringert werden.

Zur Güllebehandlung wird die erfindungsgemäße Kalk-Ton-Suspension der Güllegrube zweckmäßigerweise einige Wochen vor bzw. unmittelbar vor der Ausbringung der Gülle homogen zugemischt. Der Zusatz erfolgt zweckmäßigerweise unter ständigem Rühren.

Die zugesetzte Menge hängt dabei vom Trockensubstanzgehalt der Gülle ab. Zweckmäßigerweise werden der Gülle pro 1 Gew.-% Trockensubstanz mindestens 0,5 Gew.-% Kalk-Ton-Suspension zugesetzt, d.h. pro 1 Gew.-% Trockensubstanz 5 kg Kalk-Ton-Suspension/m³. Einer Gülle mit 8 Gew.-% Trockensubstanz werden danach z.B. etwa 40 kg Kalk-Ton-Suspension, und einer Gülle mit 5 Gew.-% Trockensubstanz ca. 25 kg Kalk-Ton-Suspension pro m³ zugemischt (berechnet für eine Kalk-Ton-Suspension mit ca. 50 Gew.-% Trockensubstanz).

Die Konzentration kann aber auch höher oder niedriger sein; die Menge an zuzusetzender Kalk-Ton-Suspension hängt dabei stark von der Zusammensetzung der Gülle ab, und insbesondere von ihrem Gehalt an Trockensubstanz.

Ein Betrieb mit einem Gülleanfall von 1200 m³ mit einem Trockensubstanzgehalt von 8 Gew.-% sollte demnach zweckmäßigerweise pro Jahr ca. 48 Tonnen einsetzen; diese Menge sollte, aufgeteilt in zwei etwa gleich großen Teilmengen, an zwei Terminen (Ausbringung im Frühjahr und Sommer/Herbst) erfolgen. Das Zumischen sollte in einem Zeitraum von einigen Wochen vor der Ausbringung erfolgen. Bereits nach der ersten Zugabe setzt ein sichtbarer Fermentierungsprozeß ein, der die Gülle geruchsärmer, fließfähiger und pflanzenverträglicher macht.

Die durch den Zusatz der erfindungsgemäßen Kalk-Ton-Suspension erzielten Wirkungen lassen sich sowohl dem Calciumcarbonat als auch den Silicaten und Tonmineralien zuschreiben.

Mehrschicht-Tonminerale haben eine katalytische Wirkung, fördern die Fermentierung, binden schädliche Stoffe und können Ammoniumstickstoff in ihre Schichtstruktur einbauen.

Durch den hohen Anteil an Kalk, der in der zugesetzten erfindungsgemäßen Kalk-Ton-Suspension enthalten ist, wird die Gülle zum Volldünger. Eine so behandelte Gülle wirkt bodenverbessernd und fördert auf dem Grünland wertvolle Gräser, Kräuter und Leguminosen.

Das nachfolgende Beispiel soll die Erfindung näher erläutern, ohne sie darauf zu beschränken. Wenn nicht anders angegeben, beziehen sich Prozentangaben (%) auf Gewichtsprozente.

### BEISPIEL

### Wirkung eines Zusatzes einer erfindungsgemäßen Kalk-Ton-Suspension zu Rindergülle auf die Fließfähigkeit und pflanzenbauliche Veränderungen

Die verwendet Gülle stammte aus einem Milchviehbetrieb und wies einen Trockensubstanzgehalt von 8,65 % auf. Dieser Gülle wurden zu unterschiedlichen Zeitpunkten zwischen 4 und 8 % der erfindungsgemäßen Kalk-Ton-Suspension zugesetzt (40 bis 80 kg Suspension/m³ Gülle).

Nach einer Lagerdauer von 12 Wochen wurde die Fließfähigkeit und die pflanzenbauliche Wirkung gemessen.

Das Fließverhalten wurde in einem Auslauftest (mit Rühren; der Auslauf besaß einen Durchmesser von 14 mm) bestimmt (Methode der ARGE-Güllebehandlung).

Die Ergebnisse der Auslauftests mit Rühren sind in Tabelle I zusammengestellt. (KTS = erfindungsgemäße Kalk-Ton-Suspension)

**TABELLE I**

| Auslauftest mit Rühren | | | | | |
|---|---|---|---|---|---|
| Auslauf Ø 14 mm | ohne Zusatz | Standardzusatz | + 4 % KTS | + 8 % KTS | + 2 % KTS |
| | 1 | 2 | 3 | 4 | 5 |
| Gesamt-Auslaufmenge in g | 8318 | 10909 | 11381 | 11611 | 10,708 |
| Nettoinhalt bei Auslagerung in g | 11940 | 11936 | 11914 | 11939 | 11919 |
| Auslaufmenge in % innerhalb 120 s | 68 | 91⁺ | 96⁺ | 97⁺ | 90⁺ |
| Ø Auslaufmenge relativ zur Variante 1 | 100 | 113 | 124 | 125 | 119 |

| | | | | | |
|---|---|---|---|---|---|
| ⁺⁾ signifikant bei α = 0,05 | | | | | |

Die pflanzenbauliche Wirkung wurde in einem Keimtest mit Kressesamen in Petrischalen auf Kunststoffiltem geprüft und nach 72 Stunden die Wurzellänge gemessen. Die Ergebnisse sind in Tabelle II zusammengestellt.

**TABELLE II**

| | Prüfgliednummer Bezeichnung | Mittlere Wurzellänge in mm relativ | Signifikanter Unterschied zu Kontrolle 1, LSD (α = 0,05) *=* 8,1 |
|---|---|---|---|
| 7 | Destilliertes Wasser | 86 | ja |
| 1 | ohne Zusatz (Kontrolle) | 100 | --- |
| 2 | Standard | 107 | nein |
| 3 | + 4 % KTS /09.06. | 118 | ja |
| 4 | + 8 % KTS /18.08. | 107 | nein |
| 5 | + 4 % KTS /09.06. + 4 % KTS /18.08. | 115 | ja |

Die vorstehenden Ergebnisse zeigen, daß durch einen Zusatz einer erfindungsgemäßen Kalk-Ton-Suspension (KTS) zu Rindergülle sowohl eine Verbesserung der Fließfähigkeit als auch der pflanzenbaulichen Wirkung (Verbesserung des Wurzelwachstums) erhalten wird.

Nach 12 Wochen Lagerdauer wurde eine Verbesserung des Fließverhaltens gegenüber der Kontrollvariante 1 bei allen Prüfvarianten in beiden Durchgängen (mit und ohne Rühren) festgestellt, und der Kressetest ergab eine Verbesserung des Wurzelwachstums gegenüber der Kontrollvariante 1. Bei den Prüfvarianten 3 und 5 sind die Ergebnisse signifikant (α = 0,05).

Durch den Zusatz der erfindungsgemäßen Kalk-Ton-Suspension wird die Gülle nicht nur aufbereitet und verbessert (Geruchsminderung, Verbesserung der Fließfähigkeit, Pflanzenverträglichkeit und pflanzenbaulichen Wirkung) sondern auch ein wesentlicher Beitrag zur Kalkversorgung (Kalkdüngung) der Böden bewirkt. Aufgrund der Basizität (der basisch wirksame CaO-Anteil beträgt vorzugsweise 15 bis 20 Gew.-%, und insbesondere ca. 18 Gew.-%) ist keine zusätzliche Kalkeinbringung mehr erforderlich, wodurch sich die Kosten der Gülleaufbereitung durch Behandeln mit der erfindungsgemäßen Kalk-Ton-Suspension nicht nur ausgleichen, sondern zum Teil sogar niedrigere Gesamtkosten erhalten werden können.

## Patentansprüche

1. Kalk-Ton-Suspension mit einem Trockensubstanzgehalt von ca. 50 Gew.-%, wobei die Trockensubstanz feinkörnig vorliegt und die Bestandteile (in Gew.-%) enthält:
| | |
|---|---|
| Calciumcarbonat (CaCO₃) | 25 - 30, |
| Magnesiumcarbonat (MgCO₃) | 0,5 - 2, |
| Siliciumoxid (SiO₂) | 8 - 12, |
| Aluminiumoxid (Al₂O₃) | 2 - 4, |
| Eisenoxid (Fe₂O₃) | 1-3. |

2. Kalk-Ton-Suspension nach Anspruch 1, **dadurch gekennzeichnet, daß** 80 Gew.-% der Trockensubstanz eine Korngröße (Siebanalyse) von 0,001 bis 0,125 mm zeigen.

3. Kalk-Ton-Suspension nach Anspruch 1 oder 2, **dadurch gekennzeichnet, daß** sie einen pH-Wert von 7,5 besitzt.

4. Kalk-Ton-Suspension nach einem der Ansprüche 1 bis 3, **dadurch gekennzeichnet, daß** sie ein Produkt aus der Verarbeitung von natürlichem Kalkstein ist.

5. Verfahren zur Herstellung einer Kalk-Ton-Suspension nach einem der Ansprüche 1 bis 4 aus einem natürlichen Kalkstein-Mineralgemisch, wobei man aus dem Mineralgemisch die abschlämmbaren Kalk-, Silicat- und Tonpartikel gewinnt und die so erhaltene Aufschlämmung zu einer Kalk-Ton-Suspension mit einem Trockensubstanzgehalt von ca. 50 Gew.-% eindickt.

6. Verwendung einer Kalk-Ton-Suspension nach einem der Ansprüche 1 bis 4 oder einer nach dem Verfahren von Anspruch 5 hergestellten Kalk-Ton-Suspension in der Bauwirtschaft, im Landschaftsbau, in der Forstwirtschaft, in der Wasserwirtschaft und in der Landwirtschaft.

7. Verwendung nach Anspruch 6 in der Landwirtschaft zur Gülleaufbereitung und Bodenverbesserung.

8. Verwendung nach Anspruch 6 zur Aufbereitung/Neutralisation von sauren Gewässern.

9. Kalk-Ton-Suspension zur Gülleaufbereitung mit einem Trockensubstanzgehalt von ca. 50 Gew.-%, wobei die Trockensubstanz feinkörnig vorliegt und die Bestandteile (in Gew.-%) enthält:
| | |
|---|---|
| Calciumcarbonat (CaCO₃) | 25 - 30, |
| Magnesiumcarbonat (MgCO₃) | 0,5 - 2, |
| Siliciumoxid (SiO₂) | 8 - 12, |
| Aluminiumoxid (Al₂O₃) | 2 - 4, |
| Eisenoxid (Fe₂O₃) | 1 -3. |

10. Kalk-Ton-Suspension nach Anspruch 9, **dadurch gekennzeichnet, daß** 80 Gew.-% der Trockensubstanz eine Korngröße (Siebanalyse) von 0,001 bis 0,125 mm zeigen.

11. Kalk-Ton-Suspension nach Anspruch 9 oder 10, **dadurch gekennzeichnet, daß** sie einen pH-Wert von 7,5 besitzt.

12. Kalk-Ton-Suspension nach einem der Ansprüche 9 bis 11, **dadurch gekennzeichnet, daß** sie ein Produkt aus der Verarbeitung von natürlichem Kalkstein ist.

13. Kalk-Ton-Suspension nach einem der Ansprüche 9 bis 12, hergestellt aus einem natürlichen Kalkstein-Mineralgemisch, wobei man aus dem Mineralgemisch die abschlämmbaren Kalk-, Silicat- und Tonpartikel gewinnt und die so erhaltene Aufschlämmung zu einer Kalk-Ton-Suspension mit einem Trockensubstanzgehalt von ca. 50 Gew.-% eindickt.
